# EUROPÄISCHE PATENTSCHRIFT

(11) **EP 0 607 777 B1**
(45) Veröffentlichungstag und Bekanntmachung des Hinweises auf die Patenterteilung: **09.12.1998**
(21) Anmeldenummer: 94100017.6
(22) Anmeldetag: 03.01.1994
(51) Int. Cl.: A61K 31/42

(54) **Verwendung von Leflunomid zur Hemmung von Interleukin 8**
Use of leflunomid for the inhibition of interleukin 8
Utilisation de leflunomid pour inhiber interleukin 8

(30) Priorität: 08.01.1993 DE 4300278
(43) Veröffentlichungstag der Anmeldung: 27.07.1994
(73) Patentinhaber: HOECHST AKTIENGESELLSCHAFT, 65929 Frankfurt am Main (DE)
(72) Erfinder: Weithmann, Klaus Ulrich, Dr., D-65719 Hofheim (DE); Bartlett, Robert Ryder, Dr., D-64291 Darmstadt (DE)

(56) Entgegenhaltungen:
- EP-A- 0 013 376
- WO-A-91/17748
- IMMUNOPHARMACOLOGY Bd. 23, Nr. 2 , 1992 Seiten 105 - 116 T. GLANT ET AL. 'Immunomodulation of proteoglycan-induced progressive polyarthritis by leflunomide'
- AGENTS AND ACTIONS Bd. 32, Nr. 1-2 , 1991 Seiten 10 - 21 R. BARTLETT ET AL. 'Leflunomide (HWA 486), a novel immunomodulating compound for the treatment of autoimmune disorders and reactions leading to transplantation rejection'

## Beschreibung

Leflunomid (s. Formel, N-(4-Trifluormethylphenyl)-5-methyl-isoxazol-4-carboxamid) ist als chemische Verbindung bereits bekannt (EP 0013376, EP 0217206, US 4351841, US 4965276).

Neben den bereits offenbarten antiphlogistischen Effekten bewirkt diese Substanz auch immunomodulatorische Wirkungen die sie zur Behandlung von Autoimmunerkrankungen und Transplantatabstossungsreaktionen befähigen. Es ist auch schon bekannt, daß ein Metabolit mit der Bezeichnung N-(4-Trifluormethylphenyl)-2-cyano-3-hydroxy-crotonsäureamid) (s. Formel) für die heilenden Wirkungen von Leflunomid verantwortlich ist.

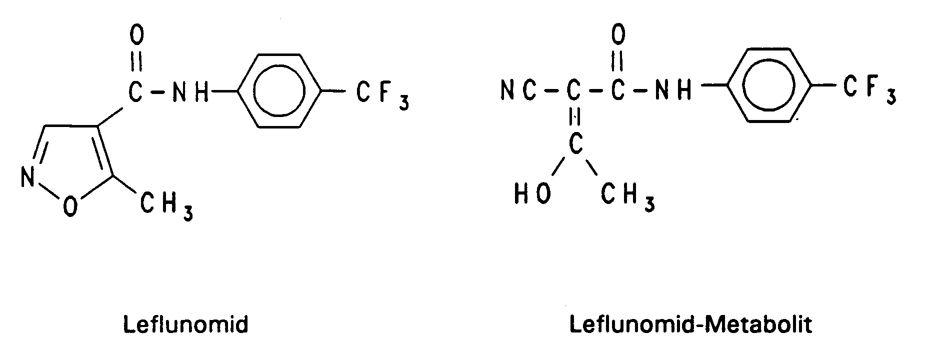

In Übereinstimmung mit dieser Erkenntnis können die oben zitierten pharmakologischen Wirkungen von Leflunomid auch durch Applikation dieses genannten Metaboliten erhalten werden (Bartlett et al, Agents and Actions, 32 (1991) 10-21).

Auch in Axton et al, J.Chem.Soc.Perkin Trans. 1 (1992) 2203ff ist beschrieben worden, daß nicht Leflunomid das aktive Prinzip darstellt, sondern daß dieser primäre Metabolit die biologischen Wirkungen entfaltet.

Sowohl in der Literatur (Bartlett et al, Agents and Actions, 32 (1991) 10-21) als auch in eigenen Experimenten konnte gezeigt werden, daß die im folgenden näher beschriebenen therapeutischen Wirkungen durch Applikation des Leflunomid-Metaboliten nicht erhalten werden können. Erfindungsgemäß wurde nämlich gefunden, daß Leflunomid einen starken inhibitorischen Einfluß auf die Synthese bzw. Freisetzung von Zytokinen aus menschlichen Blutzellen besitzt, wohingegen der Leflunomid-Metabolit diese vorteilhafte Wirkung nicht aufweist.

Unter den erfindungsgemäß angewandten experimentellen Bedingungen findet keine nennenswerte Metabolisierung des Leflunomid statt, und die inhibierende Wirkung ist ausschließlich der Substanz Leflunomid zuzuschreiben.

Bei den Zytokinen handelt es sich um eine Klasse verschiedenartiger, biologisch hochpotenter Peptide, deren Strukturen bereits bekannt sind. Es ist ebenfalls schon bekannt, daß sie endogen als Transmittersubstanzen induziert und synthetisiert werden.

Die Unterdrückung von Zytokinen im menschlichen oder tierischen Körper ist deshalb von großer medizinischer Bedeutung, weil überhöhte Spiegel dieser Zytokine zum Auftreten bzw. Ausbrechen zahlreicher Krankheiten führen können.

Zwar könnte zur Behandlung solcher Krankheiten auch ein Medikament eingesetzt werden, das die unerwünschte Wirkung des gegebenenfalls bereits vorhandenen Zytokines auf Organ-, Zell-, Gewebe- und Rezeptorsysteme des Körpers verhindert; es ist nun jedoch ein weiterer signifikanter Vorteil der vorliegenden Erfindung, daß durch Einsatz von Leflunomid bereits die Synthese bzw. Freisetzung des Zytokines verhindert wird, so daß dieses gar nicht erst entsteht, und somit die Entstehung der Krankheit bereits in einer sehr frühen Phase verhindert werden kann.

Die vorliegende Erfindung betrifft die Verwendung von Leflunomid zur Herstellung eines Arzneimittels zur Vorbeugung und Behandlung von Krankheiten des menschlichen und tierischen Körpers, die durch das Zytokin mit der Bezeichnung Interleukin 8 (IL-8) ausgelöst werden, mit Maßgabe, daß die Krankheit rheumatische Arthritis ausgeschlossen ist.

IL-8 und seine krankheitsverursachenden Wirkungen sind ausführlich in Ibelgaufts, Lexikon Zytokine, Medikon Verlag, München 1992, und in der dort zitierten Literatur beschrieben.

Wegen seiner vielfältigen biologischen Wirkungen ist IL-8 als Neutrophil-Chemotaxis-Faktor (NCF) oder Granulozyten-Chemotaxis-Peptid (GCP), aber auch als Neutrophilen aktivierender Faktor (NAF) und T-Zellen chemotaktischer Faktor (TCF) bekannt. Auch z.B. in Yoshimura et al, J. Immunol. 139 (1987) 788, Larsen et al, Science 243 (1989) 1464, Schroeder et al, J. Immunol. 139 (1987) 3474, Peveri et al, J. Exp. Med. 167 (1988) 1547, Djeu et al, J. Immunol. 144 (1990) 2205, Farina et al, Faseb J. 3 (1989) A1333, Furuta et al, J. Biochem. 106 (1989) 436, sowie in Van Damme et al, J.Exp. Med. 167 (1988) 1364 sind die vielfältigen biologischen Wirkungen von IL-8 ausführlich beschrieben. So hat dieses Zytokin ein breites Aktivitätsspektrum als Induktor von Chemotaxis und Freisetzung von Superoxid-Anionen und lysosomalen Enzymen in Neutrophil-Systemen. Auch in Basophilen und in T-Lymphozyten wird die chemotaktische Aktivität stimuliert, während die Adhäsion von Leukozyten an Endothelzellen durch IL-8 inhibiert wird. Die intraperitoneale Injektion von IL-8 führt zu Neutrophilie. Il-8-aktivierte weiße Blutzellen können vom Synovium bis in die Knorpelzellen wandern, und sind dort maßgeblich an der Entstehung von Arthritis beteiligt. Hervorzuheben ist auch, daß IL-8 schon in der Initialphase bei akuten Verwundungen, insbesondere auch schon nach kleineren Hautverletzungen im Blut nachgewiesen werden kann. Darüber hinaus wirkt es mitogen auf Epidermis-Zellen, und kann damit Hautkrebs verursachen.

Es ist somit ersichtlich, daß insbesondere IL-8 eine zentrale Stellung als Auslöser verschiedener Krankheiten und Krankheitssymptome einnimmt. Bei schwer heilenden Wunden handelt es sich überwiegend um schwerwiegende Krankheiten, deren Behandlung heute gar nicht oder nur unzureichend möglich ist. Auch deshalb kommt der aufgefundenen Wirkung von Leflunomid eine große Bedeutung zu.

Die vorliegende Erfindung betrifft ferner die Verwendung von Leflunomid zur Vorbeugung und Behandlung von Krankheiten wie, schwer heilenden Wunden (Elford and Cooper, Arthritis and Rheumatism, 34 (1991) Seite 325).

Zur Behandlung dieser Krankheiten können insbesondere auch Arneimittelformen und galenische Zubereitungen von Leflunomid, die auf üblichem Wege hergestellt worden sind, Verwendung finden.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel oder Lösungsmittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyäthylenglykole und Lösungsmittel, wie etwa sterile Wasser und ein oder mehrwertige Alkohole, z.B. Glycerin, genannt.

In der Humanmedizin werden Dosiseinheiten mit 3 bis 5 mg, vorzugsweise 10, 25 oder 50 mg pro Patient (70 kg Körpergewicht) verabfolgt. Falls medizinisch erforderlich, kann die Dosiseinheit bis auf 100, 200 oder 500 mg pro Patient gesteigert werden. Die Dosierung kann einmal täglich bis einmal wöchentlich, vorzugsweise bis zu drei oder viermal täglich, erfolgen. Die Applikation kann oral, peritoneal, intravenös, intraartikulär oder transdermal auf übliche Weise erfolgen. Aus diesen Angaben lassen sich auch leicht die entsprechenden veterinärmedizinischen Applikationen berechnen.

Schließlich kann Leflunomid bei der Herstellung der vorgenannten galenischen Zubereitungsformen auch zusammen mit anderen geeigneten Wirkstoffen, beispielsweise Antiuricopathika, Thrombocytenaggregationshemmern, Analgetika und anderen steroidalen oder nicht-steroidalen Antiphlogistika, formuliert werden.

Experimentell konnten die Wirkungen von Leflunomid an einer isolierten Blutzellfraktion (mononukleare Zellen) nachgewiesen werden, die insbesondere keine nennenswerte Metabolisierung des Leflunomids zu seinem Metaboliten bewirkte.

### Beispiel 1

Die Anreicherung der mononuklearen Zellen aus frisch gewonnenem menschlichen Zitratblut erfolgte nach bekannten Standardverfahren (s. Tiku et al, J. Immunol. 136/10 (1986) 3677):

10 ml frisch hergestelltes menschliches Zitratblut wurden vorsichtig mit 15 ml Lymphoprep^{(R)} (Molter GmbH, Heidelberg) unterschichtet und dann bei 400xg 40 min bei 20° C zentrifugiert. Die sich in der Phasengrenze als weißer Ring abzeichnende Zellfraktion wurde mit Hilfe einer Spritze entnommen, 1:1 (v/v) mit PM-16-Puffer (Fa. Serva Feinbiochemica GmbH & Co KG, Heidelberg) verdünnt und wie oben für 10 min zentrifugiert. Der Überstand wurde mit 10 ml RPMI 1640-Puffer (Gibco, Berlin), dem vorher 300 mg/l L-Glutamin zugesetzt worden waren, gewaschen. Die gewaschene Zellfraktion wurde in 1 ml RPMI 1640, dem vorher 300 mg /l L-Glutamin, 25 mmol/l HEPES (Gibco, Berlin), 0,1 g/ml Streptomyzin und 0,1 g/ml Penizyllin zugesetzt worden waren, aufgenommen. Mit Hilfe eines Zellzählers (Typ IT , Fa. Coulter Diagnostics, Krefeld) wurde die Zellsuspension, die aus ca. 90% Lymphozyten und 10% Monozyten besteht, auf etwa 5 Millionen Zellen/ml eingestellt. Die Zellviabilität wurde vor und nach den Inhibierungsexperimenten mit Hilfe der bekannten Laktatdehydrogenase-Methode überprüft. Eine Änderung der Viabilität wurde dabei nicht festgestellt.

Die Synthese und Freisetzung von zellulärem IL-8 wurde induziert, indem zu 0,48 ml der oben beschriebenen Zellfraktion eine Lösung von 500 ng Lipopolysacharid (Salmonella abortus equi, Sigma GmbH, Deisenhofen) in 0,01 ml Dimethylsulfoxid/Wasser (1:10, v/v) gegeben wurde. Gleichzeitig wurde die Zellfraktion mit einer Lösung von Leflunomid oder Leflunomid-Metabolit in 0,01 ml Dimethylsulfoxid (jeweilige Endkonzentration siehe Tab. 1) versetzt, und das Gemisch wurde für 1 h bei 37° C in einem handelsüblichen Inkubator belassen. Nach dem Abkühlen auf 0° C wurden die Proben 1 min in einer Tischzentrifuge zentrifugiert, und jeweils 0,025-ml-Aliquote des Überstandes wurden mit Hilfe des "Sandwich-" Enzymimmuno-Testkits (Fa. Biermann GmbH, Bad Nauheim) nach Herstellervorschrift spezifisch auf ihren TNFalpha-Gehalt untersucht. Die Kontrollwerte wurden ohne Zusatz von Leflunomid oder seinem Metaboliten bestimmt und auf 100% gesetzt. Insbesondere wurde durch entsprechende Vergleichs-messungen ein etwaiger Einfluß von Dimethylsulfoxid auf den TNFalpha-Spiegel ausgeschlossen.

Weiterhin wurden Aliquote des Leflunomid enthaltenden Testansatzes zeitabhängig entnommen und mit Hilfe der der Hochdruckflüssigkeitschromatographie (C-18-Säule 3,9x150 mm, Waters GmbH, Eschborn, FRG, Laufmittel: 600 ml Methanol/350 ml Wasser/ 50 ml Tetrahydrofuran/ 1 ml Phosphorsäure; Flußrate 0,7 ml/min bei 2000 pounds per square inch (Psi); Detektion im ultravioletten Bereich bei 273 nm) auf ihren Gehalt an Leflunomid oder Leflunomid-Metabolit überprüft. Es zeigte sich, daß Leflunomid unter den angewandten Bedingungen nur sehr langsam, mit einer Halbwertszeit von ca. 10 Stunden, zu seinem Metaboliten metabolisiert wird.

**TABELLE 1**

| Prüfsubstanz | Konzentration mmol/l | IL-8 im Überstand % + /- Standard-Abweichung | Anzahl Versuch n = |
|---|---|---|---|
| Leflunomid-Metabolit | | | |
| | 0,1 | 99 | 2 |
| | 0,05 | 97 | 2 |

| Leflunomid | | | |
|---|---|---|---|
| | 0,1 | 61 +/-11 | 3 |
| | 0,05 | 83 | 2 |
| ohne | 0 | 100 | |

Die Experimente in Tab. 1 zeigen, daß der Leflunomid-Metabolit praktisch keiner Einfluß auf den IL-8-Spiegel bewirkt, während der IL-8-Spiegel nach Gabe von Leflunomid deutlich gesenkt wird.

### Beispiel 2

### Herstellung von N-(4-Trifluormethylphenyl)-5-methyl-isoxazol-4-carboxamid

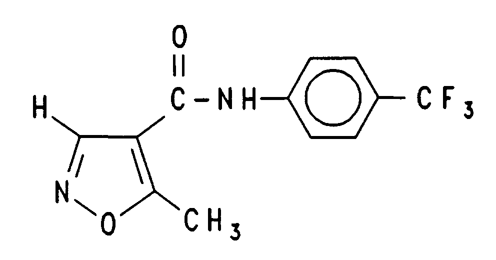

Eine Lösung von 0,05 Mol 5-Methylisoxazol-4-carbonsäurechlorid (7,3 g) in 20 ml Acetonitril werden tropfenweise bei Raumtemperatur in eine Lösung von 0,1 Mol 4-Trifluormethylanilin (16,1 g) in 150 ml Acetonitril gegeben. Nach 20 Minuten Rühren wird das ausgefallene 4-Trifluormethylanilin-hydrochlorid abgesaugt, zweimal mit je 20 ml Acetonitril gewaschen und die vereinigten Filtrate unter vermindertem Druck eingeengt. Ausbeute: 12,8 g weißes, kristallines N-(4-Trifluormethyl-phenyl)-5-methyl-isoxazol-4-carboxamid (Leflunomid).

### Beispiel 3

### Akute Toxizität nach intraperitonealer Verabreichung

Die akute Toxizität nach intraperitonealer Verabreichung der Testsubstanz wurde mit NMRI-Mäusen (20 bis 25 g) und SD-Ratten (120 bis 195 g) durchgeführt. Die Testsubstanz wurde in einer 1 %igen Natrium-CarboxymethylcelIulose-Lösung suspendiert. Die verschiedenen Dosierungen der Testsubstanz wurden den Mäusen in einem Volumen von 10 ml/kg Körpergewicht und den Ratten in einem Volumen von 5 ml/kg Körpergewicht verabreicht. Pro Dosierung wurden 10 Tiere verwendet. Nach 3 Wochen wurde die akute Toxizität nach der Methode von Lichtfield und Wilcoxon bestimmt. Die Ergebnisse sind in der Tabelle 2 zusammengefaßt:

**Tabelle 2**

| | Leflunomid akute Toxizität intraperitoneal LD₅₀ (mg/kg) |
|---|---|
| NMRI-Maus | 185 (163 - 210) |
| SD-Ratte | 170 (153 - 189) |

## Patentansprüche

1. Verwendung von N-(4-Trifluormethylphenyl)-5-methyl-isoxazol-4-carboxamid zur Herstellung eines Arzneimittels zur Vorbeugung und Behandlung von Krankheiten, die durch Interleukin 8 ausgelöst werden, mit der Maßgabe, daß die Krankheit rheumatische Arthritis ausgeschlossen ist.

2. Verwendung gemäß Anspruch 1, zur Vorbeugung und Behandlung von schwer heilenden Wunden.

## Claims

1. The use of N-(4-trifluoromethylphenyl)-5-methylisoxazole-4-carboxamide for preparing a pharmaceutical for preventing and treating disorders which are triggered by interleukin 8, with the proviso that the disorder rheumatoid arthritis is excluded.

2. The use as claimed in claim 1, for preventing and treating wounds which do not readily heal.

## Revendications

1. Utilisation du N-(4-trifluorométhylphényl)-5-méthylisoxazole-4-carboxamide pour la fabrication d'un médicament destiné à la prévention et au traitement de maladies qui sont déclenchées par l'interleukine 8, étant entendu que la maladie polyarthrite rhumatoïde est exclue.

2. Utilisation selon la revendication 1, pour la prévention et le traitement de plaies guérissant difficilement.
